# EUROPEAN PATENT SPECIFICATION

(11) **EP 2 797 882 B1**
(45) Date of publication and mention of the grant of the patent: **30.10.2019**
(21) Application number: 12786993.1
(22) Date of filing: 15.11.2012
(51) Int. Cl.: C07C 273/04, B01J 19/00

(54) **A PROCESS FOR SYNTHESIS OF UREA AND A RELATED ARRANGEMENT FOR A REACTION SECTION OF A UREA PLANT**
VERFAHREN ZUR SYNTHESE VON HARNSTOFF UND ZUGEHÖRIGE ANORDNUNG FÜR EINEN REAKTIONSABSCHNITT EINER HARNSTOFFANLAGE
PROCÉDÉ DE SYNTHÈSE D'URÉE ET AGENCEMENT ASSOCIÉ POUR UNE SECTION DE RÉACTION D'UNE INSTALLATION DE PRODUCTION D'URÉE

(30) Priority: 05.12.2011 EP 11192011
(43) Date of publication of application: 05.11.2014
(73) Proprietor: Casale SA, 6900 Lugano (CH)
(72) Inventor: SIOLI, Giancarlo, I-22010 Cernobbio (CO) (IT); CAVUOTI, Giacomo, CH-6900 Lugano (CH)
(74) Representative: Zardi, Marco
(86) International application number: PCT/EP2012/072669
(87) International publication number: WO 2013/083378

(56) References cited:
- EP-A1- 1 876 171
- US-A- 3 105 093
- US-A- 3 284 537
- US-A- 4 210 600

## Description

### Field of the invention

The invention relates to conversion of ammonia and carbon dioxide into urea. The invention relates more in detail to a novel process and arrangement for the reaction section of a urea plant.

### Prior Art

Urea is formed by reaction of ammonia with carbon dioxide, according to consecutive equilibrium reactions:

2 NH₃ + CO₂ ↔ NH₄⁺ + NH₂-COO⁻ ↔ NH₂-CO-NH₂ (urea) + H₂O

Formation of urea then involves a fast and strongly exothermic reaction between ammonia and carbon dioxide bringing to ammonium carbamate, and a slower, slightly endothermic reaction of ammonium carbamate forming urea and water. The second and slower reaction constitutes the rate-determining step of the overall chemical synthesis.

Early processes for synthesis of urea were operated at about 400 bar, with a reactor structured as a simple vertical cylindrical pressure vessel. These processes were able to achieve a good CO₂ conversion to urea (up to 80%), but suffered a low efficiency in the recovery of non-reacted NH₃ and CO₂ and practical drawbacks due to the very high pressure. Introduction of total recovery of non-converted chemicals allowed accepting lower CO₂ conversion rates (64-70%), while reducing the operating pressure down to 200-250 bar. Reducing the pressure has, of course, significant advantages in terms of the cost of the pressure vessels and other equipments, as well as energy demand for pumps and compressors.

In the known art, the above sequence of reactions is carried out by feeding NH₃ and CO₂ at the bottom section of a vertical reactor usually having a large height to diameter ratio. The largely exothermal reaction between the NH₃ and CO₂ feed and formation of ammonium carbamate takes place substantially in the lower section of the reactor, while the endothermal, slower formation of urea takes place in an upper part of the reactor. The reaction products are then crossed by an up-flow of co-current, reacting gas and liquid phases.

The conversion rate which is reached inside the reactor is substantially conditioned by mass transfer rates, in competition with rates of the chemical reactions. A urea synthesis reactor is at least partially a vapour-liquid heterogeneous reaction system where: the vapour phase contains free CO₂, NH₃, some water and inert gases; the liquid phase mainly contains NH₃, ammonium carbamate, urea, water and some ammonium carbonate. The reactants are progressively transferred from the vapour to the liquid phase, wherein CO₂ reacts with NH₃ to form the ammonium carbamate, and successively urea and water. As a consequence of the diffusion rates, chemical equilibria tend to establish, at the vapour-liquid interface, between CO₂, NH₃, H₂O in the gaseous phase and, respectively, dissolved in the liquid phase.

Attempts to improve the conversion rate have focused inter alia on the design of the reactor. For example, the provision of internal perforated plates, dividing the reactor into compartments, yielded significant conversion improvements. In this respect, US 5,304,353 discloses a reactor operating with the insertion of contact plates; US 5,750,080 discloses a method for in-situ modernisation of a reactor provided with internal, perforated plates, consisting in the addition of structurally independent caps, achieving a better gas-liquid intermixing situation; US 6,120,740 discloses reactor plates where perforations are arranged in a way to better control the liquid flow, increasing the reactor yield with the result of reducing the need to recycle non-reacted products. The latest reactors, provided with specially designed internal plates, operate the reaction at 140-160 bar, with CO₂ conversion in the range 58-62%.

Hence, it can be stated that for a given set of parameters including temperature, pressure, residence time, NH₃/CO₂ mole ratio and H₂O/CO₂ mole ratio, the efficiency of the reactor is also strongly influenced by the reactor internal design. For example, the installation of internal perforated plates is improving the gas-liquid contact, and hampering, at least partially, the internal back-mixing of products with reactants.

However, there are some drawbacks which have not yet been completely solved, including the dangerous back-mixing of products with reactants which affects the conversion rate; moreover there is an ongoing incentive and a substantial interest to seek a design capable of further raising the conversion rate to urea without increasing, or even decreasing, the pressure level.

### Summary of the Invention

The problem underlying the present invention is to improve the efficiency of the known process for urea production by acting on the configuration of the reactor or reaction section of a urea plant.

The above problem is solved with a process for synthesis of urea from reaction of ammonia and carbon dioxide, characterized in that:
- ammonia and carbon dioxide are reacted in a liquid phase and in a first reaction zone, and heat is withdrawn from said first reaction zone to promote the formation of ammonium carbamate, said first reaction zone producing a first liquid product mainly comprising ammonium carbamate, ammonia and water;
- said first product is then passed to a second reaction zone distinguished from said first reaction zone, and heat is added to said second reaction zone to promote the decomposition of ammonium carbamate into urea and water, said second reaction zone producing a second liquid product containing urea, residual unconverted carbamate and excess ammonia, and
- the liquid phase in at least one of said first reaction zone and second reaction zone being kept in a stirred condition.

The term of second reaction zone distinguished from said first reaction zone shall be understood in the sense that a reaction section of a urea plant for the above process includes a well recognizable zone (the first reaction zone) dedicated to the formation of ammonium carbamate, and a well recognizable zone (the second zone) dedicated to formation of urea. The first zone and second zone may be separated by a physical boundary, although this is not mandatory. In some embodiments the first zone and second zone are in different pressure vessels, e.g. in a first and second vessel, thus being physically separated. In some other embodiments the first zone and second zone may be arranged inside the same vessel, e.g. being an upper part and a lower part of an elongated vertical vessel.

The stirred condition shall be understood as a mechanical agitation, which is induced by rotary means. Suitable means include turbines, impellers or the like. In a preferred embodiment, said stirred condition for the first and/or the second reaction zone is provided in a fully-baffled condition of the liquid phase. Definition of the fully-baffled condition will be given below.

The invention discloses to carry out the conversion of ammonia and carbon dioxide into urea with a step-wise advancement in a series of reaction zones. The invention provides a separation between two reaction zones where the fast, exothermic formation of carbamate and, respectively, the slower endothermic dissociation into urea and water are promoted. By withdrawing heat from the first reaction zone, the formation of carbamate is promoted whilst the endothermic formation of urea is not favoured, hence a first product is substantially a solution of ammonium carbamate, ammonia and water; vice-versa, the formation of urea is promoted in the second reaction zone by adding heat. The stirred condition also plays an important role especially for enhancing the heat transfer to the liquid phase, and therefore promoting the reaction rate.

In a preferred embodiment, said second reaction zone has a temperature higher than the temperature of said first reaction zone. More preferably said second reaction zone has substantially the same pressure of said first reaction zone. More preferably the second reaction zone has a temperature higher than the first one, and substantially the same pressure. For example the first reaction zone is run at around 150 °C and the second reaction zone is run at around 180 °C. Preferred ranges are 120-170 °C for the first zone and 160-220 °C for the second zone. The working conditions inside the reactor are usually beyond the critical temperature and pressure of ammonia and carbon dioxide; accordingly, the liquid phase evolving in the reactor shall be understood as a mixture of liquids (e.g. ammonium carbamate, urea, water) and supercritical fluids.

In a preferred embodiment, the process involves also a third reaction zone, which can also termed a stripping zone, fed with a flow of second liquid product obtained in the second zone, and where the residual carbamate contained in said second liquid product is decomposed by means of a heat supply and optionally by means of addition of a stripping medium, releasing ammonia and carbon dioxide. More preferably the liquid phase in said third reaction zone is also kept in a stirred condition and preferably a strongly stirred condition.

More in detail, a gaseous stream containing NH₃ and CO₂ from decomposition of carbamate, plus some NH₃ excess, is obtained in said third zone, and is sent back to the first reaction zone for recovery purposes. The stripping of residual carbamate can be promoted in the stripping zone by adding heat and/or by adding a stripping medium such as carbon dioxide. Said stripping zone delivers a concentrated urea solution which is transferred to a downstream urea separation process, for removing water and possibly for recovering further amounts of ammonia and carbon dioxide from low pressure carbamate solution, according to a known technique.

Preferably, a gaseous stream comprising at least part of said ammonia and carbon dioxide, released in the third reaction zone by means of the stripping process, is fed directly in the gaseous state into said first reaction zone. This is a considerable advantage because a high-pressure condenser is no longer necessary, contrary to the prior art of urea processes like e.g. the conventional self-stripping or CO2-stripping processes, where a high-pressure condenser is deemed necessary.

Thanks to the fast, somewhat turbulent motion of the stirred liquid phase in the first reaction zone, the gaseous ammonia and CO2 entering the first zone will be brought to intimate contact with the liquid phase, recovering them by reaction to ammonium carbamate. Hence, previous condensation is no longer necessary, although it is possible in some embodiments.

Preferably, the gaseous flow of ammonia and carbon dioxide from the third zone is directed close to the stirring means operating in the first zone, for example close to the rotating blades of an impeller, to enhance the above effect.

According to several embodiments of the invention, any of the first reaction zone and second reaction zone may be arranged in a single vessel or more vessels or groups of vessels. Said embodiments could be mixed e.g. using one vessel for the first reaction zone, and a plurality of vessels for the second reaction zone. Physical separation between said reaction zones can be obtained with a partition wall, when the reaction zones are contained in the same vessel, although a separation wall is not a necessary feature.

If the first reaction zone and second reaction zone are comprised in the same vessel, preferably the first reaction zone is above the second reaction zone. In a preferred arrangement of a single-vessel embodiment, a pressure vessel has an upper zone forming the first reaction zone, a central zone forming the second reaction zone, and a bottom part forming the stripping zone.

Also the stripping zone may be included in the same, single vessel containing the first and second reaction zones as in the above example, or may be realized with a dedicated vessel or vessels. Preferably the stripping zone has a single, dedicated vessel. In preferred embodiments, the carbamate solution (liquid product from the second zone) and the stripping medium, if any, are directed close to rotating blades of an impeller or turbine working in the third zone, so to promote the stripping effect. The impeller may be surrounded by a heating coil which provides the necessary heat for the stripping process.

The stirred condition, as stated above, is preferably in accordance with the so-called fully-baffled condition of the liquid phase. A fully-baffled condition is known to a skilled person and a definition can be found in literature; to summarize, it is defined as a condition where the tangential entrainment of liquid is impeded, for example by appropriate baffles, and the cylindrically rotating vortex disappears, allowing transfer of a significant deal of power to the liquid under agitation.

Mechanical agitation is provided for example with one or more impellers. As a rough indication, the power transferred from the impellers to the liquid phase is preferably 0.2 to 2 kW per cubic meter of un-gassed liquid, more preferably 0.4 to 1.5 kW per m³. Hence impellers are preferably designed to deliver such power to the liquid phase, when they are in use.

Excess, humid gases from the total process are discharged from the first reaction zone and throttled to control the pressure of the system.

The steps of withdrawing or adding heat are performed with heat exchange means such as, for example, a coil traversed by a cooling medium or, respectively, a heating medium. The heat exchange means are preferably immersed in the liquid phase.

The above process has been found surprisingly efficient in solving the problems left by the known art. An advantage of the invention is the achievement of good momentum transfer conditions, thus favouring the progress of the chemical reactions involved. In addition, by means of a separation between the first and the second reaction zone, possibly in separate vessels or separate chambers of a vessel, the invention can reduce in a substantial manner the undesired back-mixing of products with reactants. A cascade of reactors, in particular, is able to avoid said back mixing.

A further advantage of the invention is that the first and second zone can be designed according to specific needs. For example, a single, stirred tank reactor may suffice for providing the first reaction zone dedicated to the fast exothermal reaction between NH₃ and CO₂; although one or more successive vessels may accomplish to the duty of the second zone, dedicated to the relatively slow, endothermal formation of urea. Finally a single, stirred tank vessel, may be individuated as the third zone, taking care of the gas stripping operation.

The heat exchange at process side, which is usually limiting the overall heat removal or supply to the reacting mass, is substantially enhanced by a mechanically stirred reactor configuration, reducing the extension of the heat exchange surface, and the reactor volume, in comparison to reactors of the known art, at equality of urea production rate per unit time.

The conversion degree of the carbon compound, without changing the operation temperature with respect to the know art, is also markedly increased.

An object of the invention is also a reaction section of a plant for synthesis of urea from ammonia and carbon dioxide, for carrying out the above process. In a general embodiment, the reaction section includes:
- a first reaction zone for conversion of ammonia and carbon dioxide into ammonium carbamate and a second reaction zone for decomposition of carbamate into urea, said second reaction zone being distinguished from said first reaction zone;
- means for feeding ammonia and carbon dioxide to said first reaction zone, and cooling means disposed in the first reaction zone and adapted to remove the heat of formation of ammonium carbamate,
- means for feeding a first product, mainly comprising ammonium carbamate, ammonia and water, from said first reaction zone to said second reaction zone;
- heating means disposed in the said second reaction zone, adapted to provide heat for the decomposition of part of said carbamate into urea, and a flow line for removing a second product containing urea, residual unconverted carbamate and excess ammonia from said second reaction zone, and
- stirring means arranged in at least one of said first reaction zone and second reaction zone and preferably in both said first and second reaction zone, the stirring means being rotary means.

Preferably the reaction section includes a third reaction zone, or stripping zone; means feeding a flow of said second liquid product from the second zone to said third zone; heating means and optionally a line for addition of a stripping medium to said third zone; stirring means for keeping the liquid phase in said third reaction zone in a stirred condition. More preferably there is provided a gas flow line for a direct connection between said third zone and first zone, arranged to recycle a gaseous flow comprising ammonia and carbon dioxide, which is released in the third reaction zone, into said first reaction zone. Even more preferably, said flow line is arranged to direct said gaseous flow close to stirring means which operates in the first reaction zone.

The reaction zones can be hosted in a single vessel, in a plurality of vessels, or multi-compartmented vessels.

A single vessel hosting the various reaction zones may be vertical or horizontal. According to a particularly preferred embodiment, the reaction zones are hosted in a single, vertical pressure vessel, and the reaction zones are arranged vertically one above the other. More preferably, fresh liquid ammonia enters the first and highest reaction zone and, hence, the reactor is traversed by the liquid stream downwards (down-flow operation). This is in contrast with the prior art, where the liquid feed enters at the bottom of the reactor, or in a lower region of the reactor.

A notable advantage of said downflow operation is that the liquid feed entering the reactor is no longer required to overcome the liquid head inside the reactor itself. In operation, a certain amount of liquid is resident in the reactor; in the prior art, the liquid feed needs to overcome the head (i.e. pressure) of said resident liquid. In the downflow-reactor embodiments of the invention, on the contrary, the liquid head inside the reactor has a positive effect and provides the motive force for feeding the effluent of the reactor to a downstream equipment, such as an external stripper or a treatment/recovery section. Thanks to the above, equipments can be placed at the same height of the reactor, instead of below the reactor, and this is a notable advantage in terms of easier installation and reduced capital costs.

Another aspect not part of the invention is method for the modernization (debottlenecking) of a vertical reactor for the synthesis of urea, where the existing reactor is converted to down-flow operation.

Some of the possible embodiments will be described below as examples. As apparent to the skilled person, other equivalent embodiments are possible, with multiple vessels, compartmented vessels or any combination thereof.

### Single-vessel embodiments

In the single-vessel embodiments of the invention, the reaction zones are hosted in a single pressure vessel. The vessel may also contain a stripping zone. More preferably, the vessel is a vertical elongated vessel and reaction zones are vertically arranged one below the other.

According to a general embodiment, a vertical reactor for the synthesis of urea from ammonia and carbon dioxide comprises a vertical pressure vessel, where:
- the pressure vessel hosts a plurality of reaction zones, including at least a first reaction zone and a second reaction zone;
- the reactor comprises stirring means arranged in at least one of said first reaction zone and second reaction zone, the stirring means being rotary means;
- the reactor also comprises first heat exchange means arranged to remove heat from said first reaction zone, and second heat exchange means arranged to furnish heat to the second reaction zone;
- said reaction zones are arranged vertically and one below the other in the pressure vessel, the first reaction zone being the highest, and are in fluid communication so that a liquid effluent from a reaction zone can flow by gravity to a reaction zone below;
- the reactor comprises a fresh liquid ammonia input line arranged to feed liquid ammonia directly in the first reaction zone, and an output for withdrawing a liquid urea effluent which is located below the second or a lower reaction zone, the reactor being then structured to operate with a liquid phase which traverses the pressure vessel downwards.

The reactor optionally includes a further reaction zone acting as a stripping zone. Carbon dioxide can be optionally fed to said stripping zone for use as a stripping medium. This stripping zone is the lowest reaction zone in the pressure vessel and comprises dedicated stirring means and heating means.

Preferably, said reactor comprises a recovery line arranged for directing a gaseous stream comprising ammonia and carbon dioxide to flow upwards in the vessel from said stripping zone to the first and upper reaction zone. Said gaseous stream may comprise carbon dioxide and ammonia coming from dissociation of carbamate, and possibly the carbon dioxide which has been added as stripping medium.

Preferably, the ammonia input line is arranged to direct the fresh liquid ammonia input in proximity of said stirring means. For example, ammonia is fed in the proximity of rotor blades of an impeller which provides the stirring of said first reaction zone. In some embodiments, also a carbon dioxide input is directed to the first reaction zone. A carbon dioxide input (if provided) is also preferably directed in the proximity of said stirring means of the first reaction zone.

The stirring means of the various reaction zones and stripping zone are preferably in the form of bladed rotors. Said rotors may be associated to a common shaft extending all along the pressure vessel. The heating or cooling means are preferably in the form of heating coils.

According to some embodiments, said vertical pressure vessel is divided into a plurality of compartments which are arranged vertically one above the other and divided by horizontal baffles. Each of said reaction zones or stripping zone is formed by one or more of said compartments. Preferably, each compartment has dedicated stirring means and heating or cooling means. For example, the upper end of the vessel constitutes the first zone, wherein the reaction of ammonia and carbon dioxide is taking place, preferably under strong agitation, and heat is removed by a cooling coil internally crossed by a cooling fluid. The mid part of the vessel is the second zone, where ammonium carbamate is left to decompose into urea and water. Heat may be supplied through a coil, to accelerate the conversion rate. The lower end of the vessel is performing, preferably under strong agitation and increased temperature, the residual carbamate decomposition and the NH₃ excess stripping. This operation may also be favoured by additional injection of CO₂ as stripping medium. Heat is preferably supplied by a heating coil, crossed by a heating fluid. The resulting gas stream may be carried up to reach the top zone, wherein it may be recovered into the carbamate formation.

### Multi-vessel embodiments

Some examples of multi-vessel embodiments are presented below.

In a first case, each reaction zone and, if provided, the stripping zone, has a single dedicated vessel. Preferably, the reaction zones are hosted in two separate stirred-tank reactors arranged in cascade, namely a first reactor providing the first reaction zone, and a second reactor providing the second reaction zone.

Each reactor is preferably equipped with a mechanical agitator; the first vessel is also equipped with a cooling coil, internally crossed by a cooling fluid, while the second vessel is equipped with a heating coil crossed by a heating fluid. In operation, NH₃ and CO₂ are fed to the first reactor, wherefrom out-flowing fluids are passed to the second reactor, wherefrom the urea solution is passing to a third vessel, where the residual carbamate is decomposed, and the resulting CO₂, if desired with additional fresh CO₂, is intimately contacted with the liquid phase by means of an adequate stirrer, with the aim of stripping out the unreacted ammonia excess, which is recycled back to the first reaction vessel.

A reaction zone may also be formed by a plurality of pressure vessels. For example, an embodiment provides a cascade of stirred tank type, vertical reactors, each constituting a separate vessel. A single reactor, for example, provides the first reaction zone, while three further vertical reactors form the second reaction zone. Each reactor is equipped with an internal, mechanical agitator, and a heat exchanger, removing or supplying heat respectively in reactors of the first or second zone. Ammonia and CO₂ are fed to the first reactor, and the fluids overflow from that reactor to the first reactor of the second stage. The last reactor of the second series delivers the final product to the final decomposition and stripping unit, wherefrom the gaseous phase is recycled back to the initial reactor of the total series.

### Multi-compartmented horizontal pressure vessels

Some embodiments make use of a horizontal pressure vessel including multiple compartments in a cascade. This kind of reactor is used preferably for the second reaction zone. For example, the second reaction zone is realised by means of a horizontal reactor, providing a series of internal compartments for the second reaction zone. Said compartments are separated by internal weirs, overflowing the liquid phases from each compartment to the next one. Each compartment is equipped with a mechanical agitator; coolers are heaters are accommodated in the various compartments, following the already described criteria.

These and other embodiments will be elucidated in the following detailed description, with the help of the drawings.

### Brief Description of the Drawings

Fig. 1 is a block scheme of a process according to a preferred embodiment of the invention.
Fig.2 is a scheme of an equipment for carrying out the process, in accordance with a single-vessel embodiment.
Fig 3 is a scheme of an equipment according to a multiple-vessel embodiment, including two stirred-tank reactors and a stripper.
Fig. 4 is a scheme of an embodiment including a cascade of stirred-tank reactors, and a stripper.
Fig. 5 is a scheme of an embodiment alternative to Fig. 4, wherein the cascade of reactors for the second reaction zone is replaced by a horizontal reactor, provided with internal, mechanically stirred compartments.
Fig. 6 is a scheme of a single-vessel vertical reactor according to another embodiment of the invention, providing two reaction zones and a final stripping zone.
Fig. 7 is a cross section of the reactor of Fig. 6.

### Detailed Description of preferred embodiments of the invention

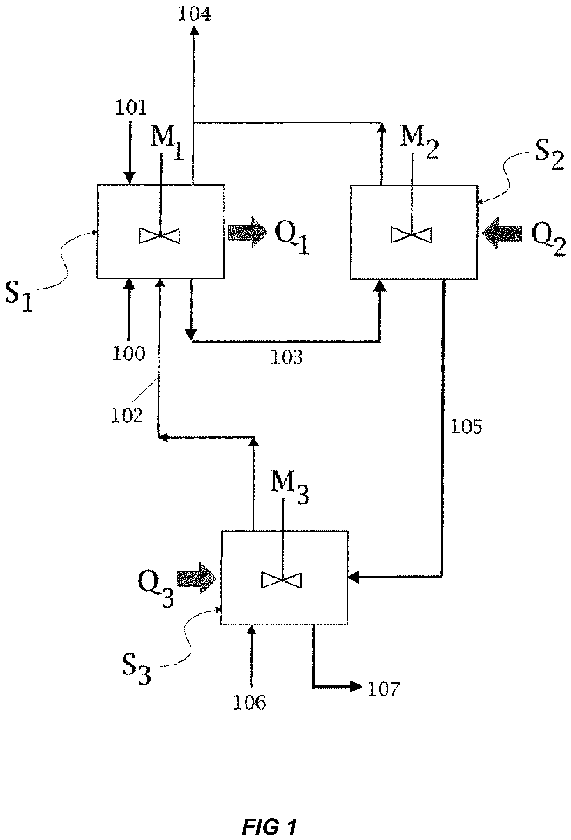

Referring to the block scheme of Fig. 1, the high-pressure conversion of carbon dioxide and ammonia into urea is carried out with a first step in a first reaction zone S1, followed by a second step in a second reaction zone S2.

A gaseous stream 100 of carbon dioxide and a liquid stream 101 containing ammonia make-up and some carbamate recycle are added to said reaction zone S1, where a liquid phase is maintained in agitation by a suitable mixer M1. A strong heat flow is released by the fast, exothermal conversion of ammonia and carbon dioxide into ammonium carbamate, and heat Q1 is removed from said reaction zone S1 to maintain the desired reaction temperature for the formation of ammonium carbamate. Heat Q1 is removed by appropriate means, e.g. by a heat exchanger crossed by a cooling medium.

The liquid phase is taken from reaction zone S1 and passed to the subsequent reaction zone S2 via line 103. The temperature of the liquid phase in the reaction zone S2 is similar or preferably higher than temperature of the liquid phase in zone S1, thus favouring the endothermic decomposition of ammonium carbamate into urea and water. This is achieved by supplying heat Q2 to zone S2 by appropriate means, e.g. a heat exchanger crossed by a heating medium.

The pressure in the second zone S2 may be substantially the same as in the first zone S1. Preferably said pressure is in the range 120 to 250 bar, more preferably around 160 bar. The liquid phase in said second zone S2 is kept in agitation by a suitable mixer M2, enhancing the transfer of heat Q2 to the liquid mass.

A concentrated aqueous solution of urea, with residual non-converted carbamate, is obtained at line 105, while a gaseous phase, mainly consisting of ammonia, carbon dioxide, water vapour and inert gases, is vented out from zones S1 and S2 via the line 104. Said line 104 may be throttled for the purpose of pressure control of the whole system.

A third reaction zone, or stripping zone, S3 is dedicated to the removal of unconverted carbamate and excess NH₃ from the reaction product 105 (urea solution), via thermal decomposition and gas stripping process. Optional addition of a stripping medium such an inert gas stream, or carbon dioxide, is indicated by line 106. The gaseous products leave said third zone S3 through the line 102, and are redirected to the first reaction zone S1, where they are partially recovered as reactants. Heat is supplied to zone S3 by appropriate means, e.g. a heat exchanger crossed by a heating medium, preferably reaching temperatures exceeding 200°C. A more concentrated urea aqueous solution is delivered by line 107. In some embodiments the redirection of gaseous products from the third zone to the first zone may require a gas compressor or a blower (not shown in the figures).

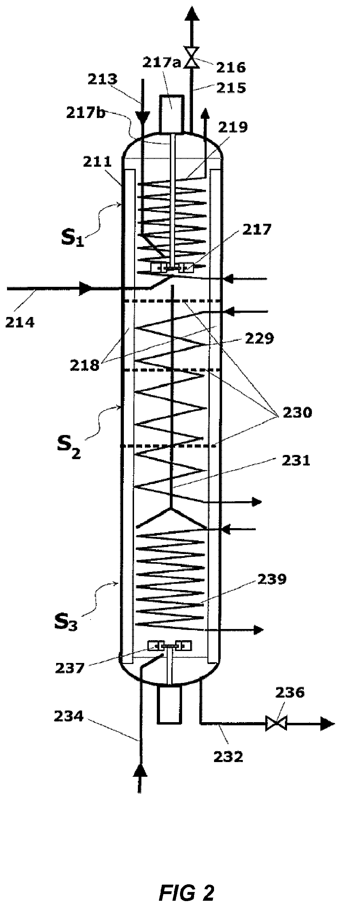

Each of the zones S1, S2 or S3 can be implemented with one or more reactor vessels. In particular, the zones S1 and S2 may be implemented with a cascade of reactors or partitioned reactors. Some preferred embodiments of the outlined technology are presented below, with reference to Fig. 2, 3 and 4.

### First embodiment

In a first embodiment of the invention, the reaction zones S1 and S2 are respectively the upper part and the mid part of a down-flow vertical reactor.

Fig. 2 shows a first implementation where the reactor is contained in a vertical, elongated pressure vessel 211 and includes: a top mixing turbine 217 and an upper heat exchange coil 219; another heat exchange coil 229 in the mid-part; perforated trays 230 and a line 231 for recovery of gaseous reactants; a bottom mixing turbine 237 and a bottom heat exchange coil 239. Baffles 218 are extended to the whole height of the vessel 211 to realize a "fully baffled" condition as explained above. The impeller 217 has a driving motor 217a and a shaft 217b extending inside the vessel 211. The mixer is preferably a magnetically-driven machine, eliminating the problem of sealing the driving shaft.

It may be noted that, in order to exploit efficiently the heat transfer conditions, in connection to the mechanical agitation, the coil assembly 219 must not prevent the liquid circulation imparted by the mixing turbine 217. Some expedients can be adopted to this purpose, as for instance by keeping the coil bank sufficiently away from the shell of the vessel 211, and by keeping a reasonable clearance between successive coils.

Ammonia, usually with some recycle of carbamate solution, is introduced via a liquid duct 213 at the top of vessel 211, in proximity of the upper face of the mixing turbine 217. Carbon dioxide is added via line 214 to the liquid phase in the vessel 211, preferably in proximity of the mixing turbine.

The product of reaction, mainly comprising carbamate, ammonia and water, flows downwards to cross the reaction zone S2. The liquid volume in S2 may be significantly larger than the volume of the first reaction zone S1, due to the relatively lower reaction rate. The heat supply from the coil 229 controls the temperature of the vessel content. The S2 zone is preferably equipped with the perforated plates 230, as used in the state of art technologies.

Finally, the liquid phase reaches the lowest part of the vessel 211 where, at higher temperature, CO₂ is evolved, and possibly added through the line 234, in proximity of the lower face of the mixer 237, with the aim of stripping out the residual excess of dissolved ammonia. The resulting gaseous stream, comprising water-saturated CO₂ and NH₃, flows up in the direction of the mixer 217, carried by the line 231, to be recovered in the upper first reaction zone S1.

The urea aqueous solution constituting the final product is available at line 232. The outflow is controlled by the valve 236, actuated on the basis of the liquid level inside the vessel. A residual gas stream is discharged from top of reactor 211 through a line 215, where a manual or automatic valve 216 controls the pressure inside the reactor itself.

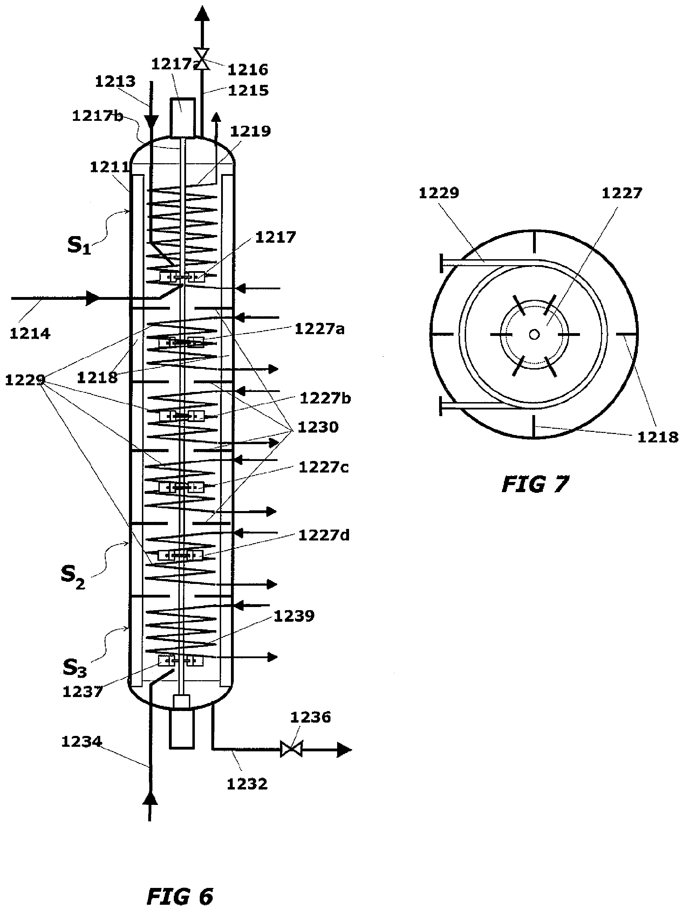

A second implementation is shown in Fig. 6. In this case, a vertical down-flow reactor is internally subdivided in a series of compartments by ring-shaped horizontal baffles 1230. One or more compartments form the reaction zones S1 or S2.

In the shown example, the first reaction zone S1 is substantially delimited by the upper compartment of the vessel 1211, above the top baffle 1230. This zone S1 is fitted with a first mixing turbine 1217 and a heat exchange coil 1219. In use, a cooling medium is circulated in said coil 1219, so that the reaction zone S1 is dedicated mainly to formation of ammonium carbamate. The second reaction zone S2 is delimited by a series of compartments below said upper compartment. Each compartment has a respective mixing turbine and heat exchanger. In the figure, the second zone S2 comprises four compartments, with the respective mixing turbines 1227a to 1227d, and heat exchange coils 1229. In use, said coils 1229 are fed with a heating medium, in order to promote the formation of urea in said zone S2. Fig. 7 shows a coil 1229 and one of said mixing turbines denoted with 1227.

The optional third reaction zone S3 is delimited by the lower compartment and is equipped with a mixing turbine 1237 and a heat exchange coil 1239. Line 1234 is an optional feed of carbon dioxide, for use as stripping medium. Preferably, said line 1234 ends in proximity of the lower face of the mixer 1237, so that the additional carbon dioxide is delivered near the blades of said mixer.

The mechanical agitation system dedicated to the full reactor comprises the driving motor 1217a and a power shaft 1217b carrying the above mentioned turbines and extending all along the vertical axis of the vessel 1211, down to a final support located at the lower end. Preferably the reactor includes longitudinal baffles 1218, extended to the whole height of the vessel, which are appropriate to realize the intensive mixing action, known as "fully baffled" condition.

Ammonia, usually with some recycle of carbamate solution, is introduced in the first zone S1 via the liquid duct 1213 from top of the vessel 1211. The end of said duct 1213 delivers the ammonia feed in proximity of the upper face of the mixing turbine 1217, operating in the upper compartment. Carbon dioxide is added via line 1214 in proximity of the lower face of the same mixing turbine. A residual gas stream is discharged through the line 1215, where the manual or automatic valve 1216 controls the pressure inside the reactor.

The products of the ammonia and carbon dioxide condensation reaction, mainly comprising carbamate, ammonia and water, obtained in the upper compartments, flow downwards to cross the compartments of the reaction zone S2 below. It should be noted that the liquid volume in S2 may be significantly larger than the volume of the first reaction zone S1, due to the relatively lower reaction rate. Coils 1229 control the temperature of the various vessel compartments in said zone S2.

Finally, the liquid phase reaches the lowest part of the vessel 1211 where, under heat supply by the coils 1239, possible residual carbamate is decomposed. Carbon dioxide evolving from decomposition of carbamate, together with carbon dioxide added through the line 1234 (if provided) in proximity of the lower face of the mixer 1237, promote the stripping out of the residual excess of dissolved ammonia. The resulting gaseous stream, comprising water-saturated CO₂ and NH₃, rises up the full length of the vessel 1211, finally reaching top compartment, near the mixer 1217. Here, the carbon dioxide and ammonia in the uprising are recovered inside the reaction zone S1.

The urea aqueous solution constituting the product of the reactor is available at line 1232. The outflow is controlled by the valve 1236, actuated on the basis of the liquid level inside the vessel.

It can be noted that the embodiment of Fig. 6 has several intermediate stirring means (mixing turbines) and has a better stage separation, compared e.g. to the simpler embodiment of Fig. 2; the latter however, might be preferred in some cases being less expensive.

### Second embodiment

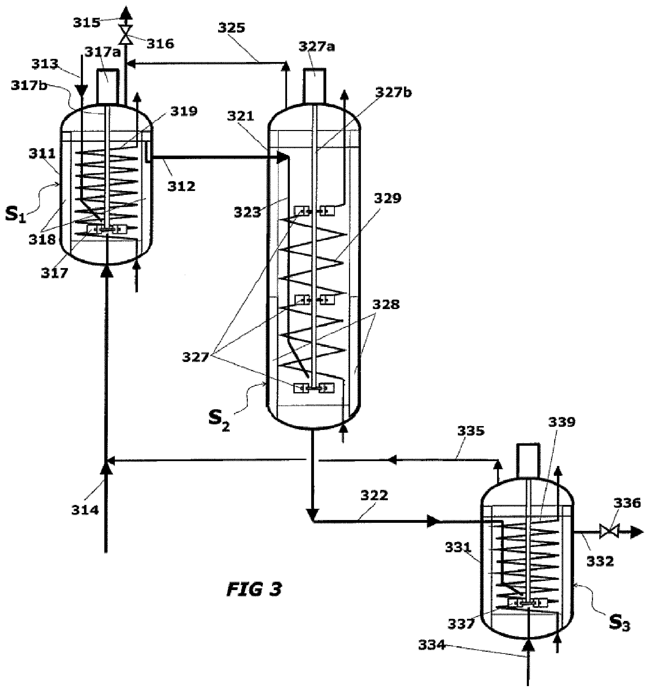

Referring to Fig. 3, reaction zones S1 and S2 are now obtained with a first stirred vessel 311 and a second stirred vessel 321, connected by a transfer line 312. A third stirred vessel 331 provides the stripping zone S3.

Vessels 311, 321 and 331 have a similar structure. They are equipped with respective mixing turbines 317, 327 and 337. References 317a, 317b, 327a, 327b denote motors and shafts. Preferably the turbines are magnetically-driven. Full-length vertical baffles 318, 328 are to realize a "fully baffled" condition

The liquid volume in S2 may be significantly larger than the volume of the first reaction zone S1, due to the relatively lower reaction rate. Due to larger volume of liquid, the second vessel 321 is usually larger than the other ones, in particular than the first vessel 311. The turbine 327 may comprise several blade sections mounted on a shaft 327b, to keep uniform agitation in said vessel 321.

The vessels also contain respective heat exchangers. In particular, a coil 319 is arranged to remove heat from the first reaction zone S1 in vessel 311, while the coils 329 and 339 supply heat to the zones S2 and S3.

Ammonia, usually with some recycle of carbamate solution, is introduced via the liquid duct 313 into the agitated vessel 311, in proximity of the upper face of said mixing turbine 317. Carbon dioxide is added via line 314 to the liquid phase in the vessel 311, in proximity of the lower face of the mixing turbine. A residual gas stream is discharged by reactor 311 through the line 315, where the manual or automatic valve 316 controls the pressure inside the reactor itself.

The reactor product, mainly comprising carbamate, ammonia and water, is collected by line 312, and transferred to the second stirred vessel 321, wherein it is released by the pipe 323 in proximity of the upper face of a mixer 327. A coil 329, located inside the vessel 321, is devised to supply heat, controlling the temperature of the vessel content.

The reactor 321 is vented together with the reactor 311 through the line 325, joining the line 315 upstream the valve 316.

The final liquid product, mainly urea in aqueous solution, is obtained at line 322 and is transferred to vessel 331. The required stripping action, necessary for the recovery of the surplus ammonia, is granted by the carbon dioxide resulting from the unconverted carbamate decomposition, with optionally added extra carbon dioxide, injected by a pipe 334 below the mixer 337. The resulting gaseous stream, comprising water-saturated CO₂ and NH₃, is transferred by the line 335, to be recovered inside the first reaction zone S1.

The urea aqueous solution, constituting the final product, is available at line 332. The outflow is controlled by the valve 336, actuated on the basis of the liquid level inside the vessel 331.

### Third embodiment

In this embodiment, the second reaction zone is set up with multiple stirred reactors, arranged in cascade or in series. The advantage is that back-mixing phenomena are minimised, in comparison to the previous embodiments, increasing the achievable conversion rate.

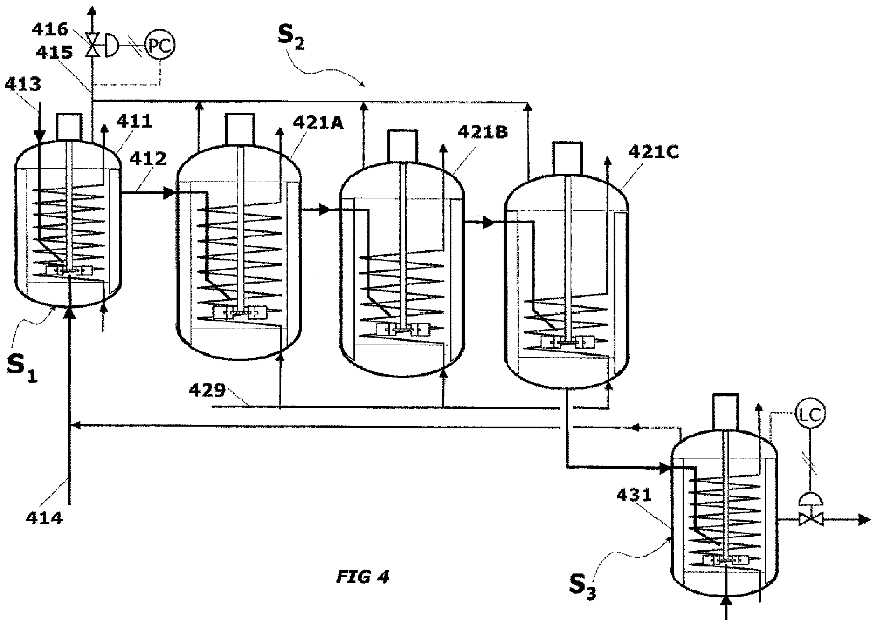

Referring to Fig. 4, the first reaction zone S1 is formed by vessel 411, while the second reaction zone S2 is formed by three vessels in cascade, items 421A, 421B and 421C. The third zone or stripping zone is in a further vessel 431. Said vessels include mixing turbines and heat exchangers similarly to embodiments of Figs. 1 to 3.

The liquid ammonia feed enters the first vessel 411 via the pipe 413, while CO₂ is fed below the mixer of the same vessel via the pipe 414. The reaction heat removal is provided by banks of coils located inside the vessel, crossed by an adequate cooling fluid. The off gas is discharged by the pipe 415, and is used to control the system pressure by means of the valve 416.

The liquid product from the first vessel 411 is transferred by pipe 412 to the reactor 421A, namely the first reactor of the cascade, and carried in proximity of the mixer thereto, as indicated by the end of flow line 412, to be evenly distributed inside the vessel.

The liquid phase, which main component is ammonium carbamate, crosses in series the cascade of stirred vessels 421A, 421B and 421C, where the decomposition of the carbamate gives out progressively urea and water. A heating medium is supplied by the pipe 429 to the coil banks of the reactors, to compensate for the required endothermic heat. The final product, aqueous urea solution with excess ammonia, is discharged from the last reactor of the cascade, say 421C, to the next stripping vessel 431. Vent lines from the cascade join the line 415, as shown.

The vessel 431 has the same duty and operating conditions as 331 in Fig.3.

### Fourth embodiment

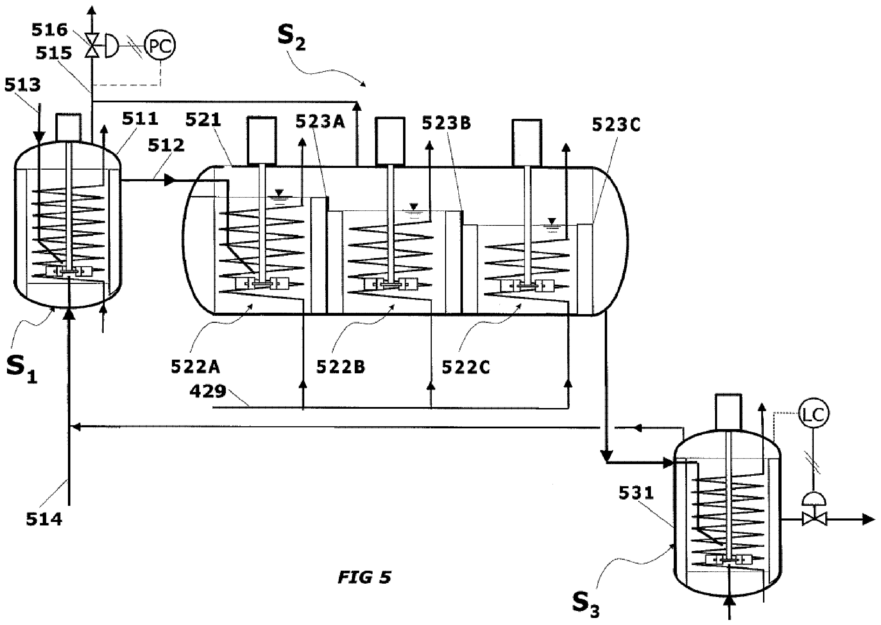

In this further embodiment, depicted in Fig.5, the second reaction zone S2 is realised by a single, multi-compartmented, horizontal vessel. A cylindrical, horizontal vessel 521 is partitioned in consecutive chambers or compartments as 522A, 522B and 522C, separated by frames 523A,523B and 523C, allowing the liquid phase to overflow from each chamber to the next one.

The first reactor vessel 511 is similar to reactors 311, 411 of the previously described embodiments. Each of the compartments in the vessel 521 has a mixing turbine and a heat exchanger.

The liquid phase from the first reactor 511, coming from line 512, crosses in series the three compartments inside the vessel 521, where urea and water are progressively obtained from the decomposition of the carbamate. A heating medium is supplied by the pipe 529 to the coil banks in the compartments 522A, 522B and 522C, to compensate for the required endothermic heat. The final product, aqueous urea solution, is discharged from the last compartment to the stripping vessel 531, as in the preceding embodiments.

### EXAMPLE

In a commercial unit, taken as a reference, producing 1000 MTPD (metric tons per day) of urea, NH₃ and CO₂ feeds, together with a carbamate recycle stream containing water, are fed into the bottom section of cylindrical, vertical reactor of 75 m³ internal volume, provided with specially perforated trays. The operating pressure is 160 bar, measured at reactor bottom section, where ammonia and recycle carbamate solution, plus gaseous CO₂, are introduced.

Under steady state conditions the reactor effluent leaves the reactor in the top section at 188°C. Said effluent is analysed in this example. The reactor material balance, based on reactor feeds, carbamate recycle solution analysis, and net urea produced, is checked as follows:

| | |
|---|---|
| urea formed in reactor | 34.2 % |
| CO₂ as unreacted carbamate | 14.7 % |
| free NH₃ plus NH₃ in carbamate | 31.3 % |
| total water | 19.8 % |

wherefrom:

| | |
|---|---|
| total CO₂ in reactor | 39.8 % |
| total NH₃ in reactor | 50.7 % |
| net water fed to reactor | 9.5 % |

and therefore:

| | |
|---|---|
| NH₃/CO₂ molar ratio | 3.30 |
| H₂O/CO₂ molar ratio | 0.58 |
| Conversion rate | 63 % |

In comparison to this commercial set up, a pilot reactor system according to a single-vessel embodiment, similar to Fig. 2 has been operated at 150 bar and 170 °C in the first reaction zone S1.

In this zone S1, heat is removed to maintain the above temperature, by circulation of pressurised water, generating low pressure steam in a separate drum. The liquid phase containing the carbamate is proceeding downwards to the zone S2, where urea is formed in practically isothermal conditions, and finally to the lower reactor end (zone S3), wherein the residual carbamate is decomposed at higher temperature (>200°C). The released CO₂ is stripping out some ammonia excess, this gaseous phase travelling upwards to the zone S1.

The resulting mass balance is as follows:

| | |
|---|---|
| urea formed in reactor | 43.5 % |
| CO₂ as unreacted carbamate | 6.7 % |
| free NH₃ plus NH₃ in carbamate | 24.8 % |
| total water | 27.0 % |

wherefrom:

| | |
|---|---|
| total CO₂ in reactor | 38.6 % |
| total NH₃ in reactor | 49.5 % |
| net water fed to reactor | 13.9 % |

therefore:

| | |
|---|---|
| NH₃/CO₂ molar ratio | 3.32 |
| H₂O/CO₂ molar ratio | 0.88 |
| Conversion rate | 82.6 % |

## Claims

1. A process for synthesis of urea from reaction of ammonia and carbon dioxide, **characterized in that**:
- ammonia and carbon dioxide are reacted in a liquid phase and in a first reaction zone (S1), and heat (Q1) is withdrawn from said first reaction zone to promote the formation of ammonium carbamate, said first reaction zone producing a first liquid product (103) mainly comprising ammonium carbamate, ammonia and water;
- said first product is then passed to a second reaction zone (S2) distinguished from said first reaction zone, and heat (Q2) is added to said second reaction zone to promote the decomposition of ammonium carbamate into urea and water, said second reaction zone producing a second liquid product (105) containing urea, residual unconverted carbamate and excess ammonia, and
- the liquid phase in at least one of said first reaction zone and second reaction zone being kept in a stirred condition induced by mechanical stirring means, the stirring means being rotary means.

2. A process according to claim 1, said stirred condition being provided in a fully-baffled condition of the liquid phase.

3. A process according to claim 1, said second reaction zone having a temperature higher than temperature of said first reaction zone, and preferably said second reaction zone having substantially the same pressure of said first reaction zone.

4. A process according to any of claims 1 to 3, said first reaction zone and said second reaction zone being physically separated,
said first reaction zone and said second reaction zone being contained in a single vessel (211, 1211) or being arranged in different vessels (311, 321; 411, 421; 511, 521) or compartments (522A - 522C) of vessels.

5. A process according to any of the preceding claims, further comprising a third reaction zone (S3), or stripping zone, fed with said second liquid product obtained in the second zone (S2), and where the carbamate contained in said second liquid product is decomposed by means of a heat supply and optionally by means of addition of a stripping medium, releasing ammonia and carbon dioxide, and the liquid phase in said third reaction zone being also kept in a stirred condition induced by mechanical stirring means, said
gaseous stream comprising at least part of said ammonia and carbon dioxide released in the third reaction zone being fed directly (102) in the gaseous state into said first reaction zone.

6. A reaction section of a urea plant, suitable for carrying out the process of claim 1, said reaction section comprising:
- a first reaction zone (S1) for conversion of ammonia and carbon dioxide into ammonium carbamate and a second reaction zone (S2) for decomposition of carbamate into urea, said second reaction zone being distinguished from said first reaction zone;
- means for feeding ammonia and carbon dioxide to said first reaction zone, and cooling means disposed in the first reaction zone and adapted to remove the heat of formation of ammonium carbamate,
- means for feeding a first product, mainly comprising ammonium carbamate, ammonia and water, from said first reaction zone to said second reaction zone;
- heating means disposed in said second reaction zone, adapted to provide heat for the decomposition of part of said carbamate into urea, and a flow line for removing a second product containing urea, residual unconverted carbamate and excess ammonia from said second reaction zone, and
- stirring means arranged in at least one of said first reaction zone and second reaction zone, the stirring means being rotary means.

7. A reaction section according to claim 6, further comprising a third reaction zone (S3), or stripping zone; means feeding a flow of said second liquid product from the second zone (S2) to said third zone (S3); heating means for heating said third zone; optionally a line for addition of a stripping medium to said third zone; stirring means for keeping the liquid phase in said third reaction zone in a stirred condition.

8. A reaction section according to claim 7, further comprising a gas flow line (102, 231, 335) for a direct connection between said third zone and first zone, arranged to recycle a gaseous flow of ammonia and carbon dioxide released in the third reaction zone into said first reaction zone,
said gas flow line being preferably arranged to direct said gaseous flow close to stirring means (217, 317) acting in the first reaction zone.

9. A reaction section according to any of claims 6 to 8, said first reaction zone and second reaction zone being hosted in a single vessel.

10. A reaction section according to any of claims 6 to 8, comprising:
- a first pressure vessel (311, 411, 511) containing the first reaction zone, and comprising a heat exchanger for cooling the first reaction zone, and a first impeller for providing a stirred condition of the liquid phase in said first reaction zone, and
- a second pressure vessel (321, 521) containing the second reaction zone or a compartmented second pressure vessel (521) comprising a cascade of compartments (522A, 522B, 522C) or a plurality of second pressure vessels (421A, 421B, 421C) arranged in a cascade, each of said compartments or second pressure vessels being a respective portion of said second reaction zone,
- said second pressure vessel comprising at least one heat exchanger for heating the second reaction zone, and at least one second impeller for providing a stirred condition of the liquid phase in said second reaction zone, or each of said compartments or second vessels having a respective heat exchanger and impeller.

11. A vertical reactor for the synthesis of urea from ammonia and carbon dioxide with the process of claim 1, comprising a vertical pressure vessel (211, 1211), where:
- the pressure vessel hosts a plurality of reaction zones, including at least a first reaction zone (S1) and a second reaction zone (S2);
- the reactor comprises stirring means (217, 1217, 1227a - 1227d) arranged in at least one of said first reaction zone and second reaction zone, the stirring means being rotary means;
- the reactor also comprises first heat exchange means (219, 1219) arranged to remove heat from said first reaction zone, and second heat exchange means (229, 1229) arranged to furnish heat to the second reaction zone;
- said reaction zones are arranged vertically and one above the other in the pressure vessel, the first reaction zone being the highest, and are in fluid communication so that a liquid effluent from a reaction zone (S1, S2) can flow by gravity to a reaction zone below (S2, S3);
- the reactor comprises a fresh liquid ammonia input line (213, 1213) arranged to feed liquid ammonia directly in the first reaction zone, and an output (232, 1232) for withdrawing a liquid urea effluent which is located below the second or a lower reaction zone, the reactor being then structured to operate with a liquid phase which traverses the pressure vessel downward.

12. A reactor according to claim 11, the pressure vessel comprising a further reaction zone (S3) which:
- is the lowest reaction zone in the pressure vessel;
- comprises dedicated stirring means (237, 1237) and heating means (239, 1239), and
- act substantially as a stripping zone;
said reactor comprising a recovery line (231) arranged for directing a gaseous stream comprising ammonia and carbon dioxide to flow upwards in the vessel from said stripping zone (S3) to the first and upper reaction zone (S1).

13. A reactor according to claim 11 or 12, said ammonia input line (213, 1213) being arranged to direct the fresh liquid ammonia input in proximity of said stirring means (217, 1217).

14. A reactor according to claim 13, comprising also a carbon dioxide input line (214, 1214), arranged to feed carbon dioxide in said first region of the pressure vessel, and preferably in proximity of said stirring means.

15. A reactor according to any of claims 11 to 14, comprising a plurality of compartments inside the pressure vessel, the compartments being arranged vertically one above the other and divided by horizontal baffles (230, 1230), wherein each of said reaction zones (S1, S2, S3) is formed by one or more of said compartments.

16. A reactor according to any of claims 11 to 15, each compartment having dedicated stirring means and heating means, and said reactor comprising:
- an upper compartment which delimits the first reaction zone (S1);
- a plurality of intermediate compartments which delimit the second reaction zone (S2);
- a lower compartment which delimits the stripping zone (S3).

## Patentansprüche

1. Verfahren zur Synthese von Harnstoff aus der Reaktion von Ammoniak und Kohlendioxid, **dadurch gekennzeichnet, dass**:
- Ammoniak und Kohlendioxid in einer flüssigen Phase und in einer ersten Reaktionszone (S1) umgesetzt werden und der ersten Reaktionszone Wärme (Q1) entzogen wird, um die Bildung von Ammoniumcarbamat zu fördern, wobei die erste Reaktionszone ein erstes flüssiges Produkt (103) erzeugt, das hauptsächlich Ammoniumcarbamat, Ammoniak und Wasser umfasst;
- das erste Produkt dann zu einer zweiten Reaktionszone (S2) geleitet wird, die sich von der ersten Reaktionszone unterscheidet, und der zweiten Reaktionszone Wärme (Q2) zugeführt wird, um die Zersetzung von Ammoniumcarbamat in Harnstoff und Wasser zu fördern, wobei die zweite Reaktionszone ein zweites flüssiges Produkt (105) erzeugt, das Harnstoff, nicht umgewandeltes Restcarbamat und überschüssiges Ammoniak enthält, und
- die flüssige Phase in der ersten Reaktionszone und/oder der zweiten Reaktionszone in einem durch mechanische Rührmittel hervorgerufenen Rührzustand gehalten wird, wobei die Rührmittel rotierende Mittel sind.

2. Verfahren nach Anspruch 1, wobei der Rührzustand in einem vollständig abgeschirmten Zustand der flüssigen Phase vorgesehen wird.

3. Verfahren nach Anspruch 1, wobei die zweite Reaktionszone eine Temperatur aufweist, die höher als die Temperatur der ersten Reaktionszone ist, und vorzugsweise die zweite Reaktionszone weitgehend den gleichen Druck wie die erste Reaktionszone hat.

4. Verfahren nach einem der Ansprüche 1 bis 3, wobei die erste Reaktionszone und die zweite Reaktionszone physisch getrennt sind,
wobei die erste Reaktionszone und die zweite Reaktionszone in einem einzigen Behälter (211, 1211) enthalten sind oder in unterschiedlichen Behältern (311, 321 ; 411, 421; 511, 521) oder in Kammern (522A - 522C) von Behältern angeordnet sind.

5. Verfahren nach einem der vorstehenden Ansprüche, weiter umfassend eine dritte Reaktionszone (S3) oder Strippzone, der das zweite flüssige Produkt zugeführt wird, das in der zweiten Zone (S2) erhalten wird, und wobei das im zweiten flüssigen Produkt enthaltene Carbamat durch eine Wärmezufuhr und wahlweise durch Zugabe eines Strippmediums unter Freisetzung von Ammoniak und Kohlendioxid zersetzt wird, und wobei die flüssige Phase in der dritten Reaktionszone ebenfalls in einem durch mechanische Rührmittel hervorgerufenen Rührzustand gehalten wird, wobei
der gasförmige Strom, der zumindest einen Teil des in der dritten Reaktionszone freigesetzten Ammoniaks und Kohlendioxids umfasst, direkt (102) im gasförmigen Zustand in die erste Reaktionszone eingeleitet wird.

6. Reaktionsabschnitt einer Harnstoffanlage, die zum Durchführen des Verfahrens nach Anspruch 1 geeignet ist, wobei der Reaktionsabschnitt umfasst:
- eine erste Reaktionszone (S1) zur Umwandlung von Ammoniak und Kohlendioxid in Ammoniumcarbamat und eine zweite Reaktionszone (S2) zur Zersetzung von Carbamat in Harnstoff, wobei sich die zweite Reaktionszone von der ersten Reaktionszone unterscheidet;
- Mittel zum Zuführen von Ammoniak und Kohlendioxid in die erste Reaktionszone und Kühlmittel, die sich in der ersten Reaktionszone befinden und geeignet sind, um die Wärme der Ammoniumcarbamatbildung abzuführen,
- Mittel zum Zuführen eines ersten Produkts, das hauptsächlich Ammoniumcarbamat, Ammoniak und Wasser umfasst, aus der ersten Reaktionszone in die zweite Reaktionszone;
- Heizmittel, die sich in der zweiten Reaktionszone befinden und geeignet sind, um Wärme für die Zersetzung eines Teils des Carbamats in Harnstoff vorzusehen, und eine Strömungsleitung zum Entfernen eines zweiten Produkts, das Harnstoff, nicht umgewandeltes Restcarbamat und überschüssiges Ammoniak enthält, aus der zweiten Reaktionszone und
- Rührmittel, die in der ersten Reaktionszone und/oder der zweiten Reaktionszone angeordnet sind, wobei die Rührmittel rotierende Mittel sind.

7. Reaktionsabschnitt nach Anspruch 6, weiter umfassend eine dritte Reaktionszone (S3) oder Strippzone; Mittel zum Zuführen eines Stroms des zweiten flüssigen Produkts aus der zweiten Zone (S2) in die dritte Zone (S3); Heizmittel zum Erwärmen der dritten Zone; wahlweise eine Leitung zur Zugabe eines Strippmediums in die dritte Zone; Rührmittel zum Halten der flüssigen Phase in der dritten Reaktionszone in einem Rührzustand.

8. Reaktionsabschnitt nach Anspruch 7, weiter umfassend eine Gasströmungsleitung (102, 231, 335) für eine direkte Verbindung zwischen der dritten Zone und der ersten Zone, die angeordnet ist, um einen gasförmigen Strom von Ammoniak und Kohlendioxid, der in der dritten Reaktionszone freigesetzt wird, in die erste Reaktionszone zurückzuführen,
wobei die Gasströmungsleitung vorzugsweise angeordnet ist, um den gasförmigen Strom in die Nähe von Rührmitteln (217, 317) zu leiten, die in der ersten Reaktionszone aktiv sind.

9. Reaktionsabschnitt nach einem der Ansprüche 6 bis 8, wobei die erste Reaktionszone und die zweite Reaktionszone in einem einzigen Behälter untergebracht sind.

10. Reaktionsabschnitt nach einem der Ansprüche 6 bis 8, umfassend:
- einen ersten Druckbehälter (311, 411, 511), der die erste Reaktionszone enthält und einen Wärmetauscher zum Kühlen der ersten Reaktionszone umfasst, und ein erstes Flügelrad zum Vorsehen eines Rührzustands der flüssigen Phase in der ersten Reaktionszone, und
- einen zweiten Druckbehälter (321, 521), der die zweite Reaktionszone oder einen unterteilten zweiten Druckbehälter (521) enthält, der eine Kaskade von Kammern (522A, 522B, 522C) oder eine Vielzahl von zweiten Druckbehältern (421 A, 421B, 421C) umfasst, die in einer Kaskade angeordnet sind, wobei jede der Kammern oder zweiten Druckbehälter ein jeweiliger Abschnitt der zweiten Reaktionszone ist,
- wobei der zweite Druckbehälter mindestens einen Wärmetauscher zum Erwärmen der zweiten Reaktionszone und mindestens ein zweites Laufrad zum Vorsehen eines Rührzustands der flüssigen Phase in der zweiten Reaktionszone umfasst oder jede der Kammern oder zweiten Behälter einen jeweiligen Wärmetauscher und ein jeweiliges Flügelrad aufweist.

11. Vertikaler Reaktor für die Synthese von Harnstoff aus Ammoniak und Kohlendioxid mit dem Verfahren nach Anspruch 1, umfassend einen vertikalen Druckbehälter (211, 1211), wobei:
- der Druckbehälter eine Vielzahl von Reaktionszonen beherbergt, die zumindest eine erste Reaktionszone (S1) und eine zweite Reaktionszone (S2) aufweisen:
- der Reaktor Rührmittel (217, 1217, 1227a - 1227d) umfasst, die in der ersten Reaktionszone und/oder zweiten Reaktionszone angeordnet sind, wobei die Rührmittel rotierende Mittel sind;
- der Reaktor auch erste Wärmetauschmittel (219, 1219), die angeordnet sind, um der ersten Reaktionszone Wärme zu entziehen, und zweite Wärmetauschmittel (229, 1229) umfasst, die angeordnet sind, um der zweiten Reaktionszone Wärme zuzuführen;
- die Reaktionszonen vertikal und übereinander im Druckbehälter angeordnet sind, wobei die erste Reaktionszone die höchste ist, und in Fluidverbindung stehen, so dass ein flüssiger Abfluss aus einer Reaktionszone (S1, S2) durch Schwerkraft zu einer darunterliegenden Reaktionszone (S2, S3) strömen kann;
- der Reaktor eine Eingangsleitung für frischen flüssigen Ammoniak (213, 1213), die angeordnet ist, um flüssigen Ammoniak direkt der ersten Reaktionszone zuzuführen, und einen Ausgang (232, 1232) zum Abziehen eines flüssigen Harnstoffabflusses umfasst, der sich unterhalb der zweiten oder einer unteren Reaktionszone befindet, wobei der Reaktor dann aufgebaut ist, um mit einer flüssigen Phase zu arbeiten, die den Druckbehälter nach unten durchquert.

12. Reaktor nach Anspruch 11, wobei der Druckbehälter eine weitere Reaktionszone (S3) umfasst, die:
- die unterste Reaktionszone im Druckbehälter ist;
- spezielle Rührmittel (237, 1237) und Heizmittel (239, 1239) umfasst, und
- im Wesentlichen als Strippzone wirkt;
wobei der Reaktor eine Rückgewinnungsleitung (231) umfasst, die angeordnet ist, um einen gasförmigen Strom, der Ammoniak und Kohlendioxid umfasst, von der Strippzone (S3) zur ersten und oberen Reaktionszone (S1) nach oben in den Behälter zu leiten.

13. Reaktor nach Anspruch 11 oder 12, wobei die Ammoniak-Eingangsleitung (213, 1213) angeordnet ist, um den frischen flüssigen Ammoniakeintrag in die Nähe der Rührmittel (217, 1217) zu leiten.

14. Reaktor nach Anspruch 13, weiter umfassend eine Kohlendioxid-Eingangsleitung (214, 1214), die angeordnet ist, um Kohlendioxid in den ersten Bereich des Druckbehälters und vorzugsweise in die Nähe der Rührmittel zu leiten.

15. Reaktor nach einem der Ansprüche 11 bis 14, umfassend eine Vielzahl von Kammern im Inneren des Druckbehälters, wobei die Kammern vertikal übereinander angeordnet und durch horizontale Leitbleche (230, 1230) unterteilt sind, wobei jede der Reaktionszonen (S1, S2, S3) durch eine oder mehrere der Kammern ausgebildet ist.

16. Reaktor nach einem der Ansprüche 11 bis 15, wobei jede Kammer spezielle Rührmittel und Heizmittel aufweist und der Reaktor umfasst:
- eine obere Kammer, die die erste Reaktionszone (S1) begrenzt;
- eine Vielzahl von Zwischenkammern, die die zweite Reaktionszone (S2) begrenzen;
- eine untere Kammer, die die Strippzone (S3) begrenzt.

## Revendications

1. Procédé pour la synthèse d'urée à partir de la réaction d'ammoniac et de dioxyde de carbone, **caractérisé en ce que** :
- de l'ammoniac et du dioxyde de carbone sont mis à réagir en phase liquide dans une première zone réactionnelle (S1), et la chaleur (Q1) est retirée de ladite première zone réactionnelle pour que la formation de carbamate d'ammonium soit favorisée, ladite première zone réactionnelle produisant un premier produit liquide (103) comprenant principalement du carbamate d'ammonium, de l'ammoniac et de l'eau ;
- ledit premier produit passe ensuite dans une deuxième zone réactionnelle (S2) distincte de ladite première zone réactionnelle, et de la chaleur (Q2) est ajoutée à ladite deuxième zone réactionnelle pour que la décomposition du carbamate d'ammonium en urée et eau soit favorisée, ladite deuxième zone réactionnelle produisant un deuxième produit liquide (105) contenant de l'urée, du carbamate non converti résiduel et de l'ammoniac en excès, et
- la phase liquide dans au moins l'une parmi lesdites première zone réactionnelle et deuxième zone réactionnelle est maintenue dans un état agité induit par des moyens d'agitation mécaniques, les moyens d'agitation étant des moyens rotatifs.

2. Procédé selon la revendication 1, dans lequel ledit état agité est assuré dans un état entièrement chicané de la phase liquide.

3. Procédé selon la revendication 1, dans lequel ladite deuxième zone réactionnelle a une température supérieure à la température de ladite première zone réactionnelle, et de préférence ladite deuxième zone réactionnelle a pratiquement la même pression que ladite première zone réactionnelle.

4. Procédé selon l'une quelconque des revendications 1 à 3, dans lequel ladite première zone réactionnelle et ladite deuxième zone réactionnelle sont séparées physiquement,
ladite première zone réactionnelle et ladite deuxième zone réactionnelle étant contenues dans un seul récipient (211, 1211) ou étant disposées dans des récipients différents (311, 321 ; 411, 421 ; 511, 521) ou des compartiments différents (522A - 522C) de récipients.

5. Procédé selon l'une quelconque des revendications précédentes, comprenant en outre une troisième zone réactionnelle (S3), ou zone d'extraction, alimentée en ledit deuxième produit liquide obtenu dans la deuxième zone (S2), et où le carbamate contenu dans ledit deuxième produit liquide est décomposé au moyen d'un apport de chaleur et éventuellement au moyen de l'addition d'un milieu d'extraction, libérant de l'ammoniac et du dioxyde de carbone, et la phase liquide dans ladite troisième zone réactionnelle étant également maintenue dans un état agité induit par des moyens d'agitation mécaniques, ledit courant gazeux, qui comprend au moins une partie dudit ammoniac et dudit dioxyde de carbone libérés dans la troisième zone réactionnelle, étant introduit directement (102) à l'état gazeux dans ladite première zone réactionnelle.

6. Section réactionnelle d'une usine d'urée, convenant pour la mise en œuvre du procédé de la revendication 1, ladite section réactionnelle comprenant :
- une première zone réactionnelle (S1) pour la conversion d'ammoniac et de dioxyde de carbone en carbamate d'ammonium et une deuxième zone réactionnelle (S2) pour la décomposition de carbamate en urée, ladite deuxième zone réactionnelle étant distincte de ladite première zone réactionnelle ;
- des moyens pour introduire de l'ammoniac et du dioxyde de carbone dans ladite première zone réactionnelle, et des moyens de refroidissement disposés dans la première zone réactionnelle et adaptés pour éliminer la chaleur de formation du carbamate d'ammonium,
- des moyens pour introduire un premier produit, comprenant principalement du carbamate d'ammonium, de l'ammoniac et de l'eau, depuis ladite première zone réactionnelle dans ladite deuxième zone réactionnelle ;
- des moyens de chauffage disposés dans ladite deuxième zone réactionnelle, adaptés pour apporter de la chaleur pour la décomposition d'une partie dudit carbamate en urée, et une ligne d'écoulement pour retirer un deuxième produit contenant de l'urée, du carbamate non converti résiduel et de l'ammoniac en excès hors de ladite deuxième zone réactionnelle, et
- des moyens d'agitation disposés dans au moins l'une parmi lesdites première zone réactionnelle et deuxième zone réactionnelle, les moyens d'agitation étant des moyens rotatifs.

7. Section réactionnelle selon la revendication 6, comprenant en outre une troisième zone réactionnelle (S3), ou zone d'extraction ; des moyens pour introduire un courant dudit deuxième produit liquide depuis la deuxième zone (S2) dans ladite troisième zone (S3) ; des moyens de chauffage pour chauffer ladite troisième zone ; éventuellement une ligne pour l'addition d'un milieu d'extraction dans ladite troisième zone ; des moyens d'agitation pour maintenir la phase liquide dans ladite troisième zone réactionnelle dans un état agité.

8. Section réactionnelle selon la revendication 7, comprenant en outre une ligne d'écoulement de gaz (102, 231, 335) pour une connexion directe entre lesdites troisième zone et première zone, disposée de manière à recycler un écoulement gazeux d'ammoniac et de dioxyde de carbone, libéré dans la troisième zone réactionnelle, dans ladite première zone réactionnelle,
ladite ligne d'écoulement de gaz étant de préférence disposée de manière à diriger ledit écoulement gazeux à proximité des moyens d'agitation (217, 317) agissant dans la première zone réactionnelle.

9. Section réactionnelle selon l'une quelconque des revendications 6 à 8, dans laquelle lesdites première zone réactionnelle et deuxième zone réactionnelle sont disposées dans un seul récipient.

10. Section réactionnelle selon l'une quelconque des revendications 6 à 8, comprenant :
- un premier récipient sous pression (311, 411, 511) contenant la première zone réactionnelle, et comprenant un échangeur de chaleur pour refroidir la première zone réactionnelle, et un premier agitateur à hélice pour réaliser un état agité de la phase liquide dans ladite première zone réactionnelle, et
- un deuxième récipient sous pression (321, 521) contenant la deuxième zone réactionnelle ou un deuxième récipient sous pression compartimenté (521) comprenant une cascade de compartiments (522A, 522B, 522C) ou une pluralité de deuxièmes récipients sous pression (421A, 421B, 421C) disposés en cascade, chacun desdits compartiments ou deuxièmes récipients sous pression étant une partie respective de ladite deuxième zone réactionnelle,
- ledit deuxième récipient sous pression comprenant au moins un échangeur de chaleur pour chauffer la deuxième zone réactionnelle, et au moins un deuxième agitateur à hélice pour réaliser un état agité de la phase liquide dans ladite deuxième zone réactionnelle, ou bien chacun desdits compartiments ou deuxièmes récipients ayant un échangeur de chaleur et un agitateur à hélice respectifs.

11. Réacteur vertical pour la synthèse d'urée à partir d'ammoniac et de dioxyde de carbone avec le procédé de la revendication 1, comprenant un récipient sous pression vertical (211, 1211), dans lequel :
- le récipient sous pression contient une pluralité de zones réactionnelles, y compris au moins une première zone réactionnelle (S1) et une deuxième zone réactionnelle (S2) ;
- le réacteur comprend des moyens d'agitation (217, 1217, 1227a - 1227d) disposés dans au moins l'une parmi lesdites première zone réactionnelle et deuxième zone réactionnelle, les moyens d'agitation étant des moyens rotatifs ;
- le réacteur comprend aussi des premiers moyens d'échange de chaleur (219, 1219) disposés de manière à retirer la chaleur de ladite première zone réactionnelle, et des deuxièmes moyens d'échange de chaleur (229, 1229) disposés de manière à fournir de la chaleur à la deuxième zone réactionnelle ;
- lesdites zones réactionnelles sont disposées verticalement et les unes au-dessus des autres dans le récipient sous pression, la première zone réactionnelle étant la plus élevée, et sont en communication de fluide de façon qu'un effluent liquide provenant d'une zone réactionnelle (S1, S2) puisse s'écouler par gravité dans une zone réactionnelle située dessous (S2, S3) ;
- le réacteur comprend une ligne d'entrée d'ammoniac liquide frais (213, 1213) disposée de manière à introduire de l'ammoniac liquide directement dans la première zone réactionnelle, et une sortie (232, 1232) pour soutirer un effluent d'urée liquide qui est située sous la deuxième zone réactionnelle ou une zone réactionnelle inférieure, le réacteur étant ensuite structuré de manière à fonctionner avec une phase liquide qui traverse le récipient sous pression vers le bas.

12. Réacteur selon la revendication 11, dans lequel le récipient sous pression comprend une autre zone réactionnelle (S3) qui :
- est la zone réactionnelle la plus basse dans le récipient sous pression ;
- comprend des moyens d'agitation dédiés (237, 1237) et des moyens de chauffage (239, 1239), et
- agit sensiblement comme une zone d'extraction ; ledit réacteur comprenant une ligne de récupération (231) disposée de manière à diriger un courant gazeux comprenant de l'ammoniac et du dioxyde de carbone pour qu'il s'écoule vers le haut dans le récipient depuis ladite zone d'extraction (S3) dans la première zone réactionnelle supérieure (S1).

13. Réacteur selon la revendication 11 ou 12, dans lequel ladite ligne d'entrée d'ammoniac (213, 1213) est disposée de manière à diriger l'entrée d'ammoniac liquide frais à proximité desdits moyens d'agitation (217, 1217).

14. Réacteur selon la revendication 13, comprenant aussi une ligne d'entrée de dioxyde de carbone (214, 1214), disposée de manière à introduire du dioxyde de carbone dans ladite première région du récipient sous pression, et de préférence à proximité desdits moyens d'agitation.

15. Réacteur selon l'une quelconque des revendications 11 à 14, comprenant une pluralité de compartiments à l'intérieur du récipient sous pression, les compartiments étant disposés verticalement les uns au-dessus des autres et étant divisés par des déflecteurs horizontaux (230, 1230), dans lequel chacune desdites zones réactionnelles (S1, S2, S3) est formée d'un ou plusieurs desdits compartiments.

16. Réacteur selon l'une quelconque des revendications 11 à 15, dans lequel chaque compartiment a des moyens d'agitation et des moyens de chauffage dédiés, et ledit réacteur comprenant :
- un compartiment supérieur qui délimite la première zone réactionnelle (S1) ;
- une pluralité de compartiments intermédiaires qui délimitent la deuxième zone réactionnelle (S2) ;
- un compartiment inférieur qui délimite la zone d'extraction (S3).
